# EUROPEAN PATENT APPLICATION

(11) **EP 3 150 118 A1**
(43) Date of publication of application: **05.04.2017**
(21) Application number: 14893261.9
(22) Date of filing: 30.05.2014
(51) Int. Cl.: A61B 5/0408, A61B 5/053, A61N 1/04

(54) **MODULE FOR DETECTING BODILY SIGNALS**

(71) Applicant: Holzhacker, Rafael, CEP: 01456-030 São Paulo, SP (BR)
(72) Inventor: Holzhacker, Rafael, CEP: 01456-030 São Paulo, SP (BR)
(74) Representative: Schwabe - Sandmair - Marx
(86) International application number: PCT/BR2014/000181
(87) International publication number: WO 2015/179933

(57) **Abstract**

The present invention refers to a module (1) to capture signals from the body of a patient (animal or human), comprising at least one sensor to capture cardiac electrical signals (4) and at least one sensor to capture body impedance signals (5). This invention also refers to a module (1) to capture signals from the body of a patient (animal or human), comprising at least one sensor (8) capable of simultaneously capturing cardiac electrical signals and/or body impedance signals.

## Description

### PRIORITY CLAIM

This application is a national phase entry under 35 U.S.C. § 371 of International Patent Application PCT/BR2014/000181, filed May 30, 2014, designating the United States of America and published as International Patent Publication WO 2015/179933 A1 on December 3, 2015.

### TECHNICAL FIELD

This invention refers to a module to capture body signals of a patient (human or animal), such as cardiac electrical signals and body impedance signals.

### BACKGROUND

Electrical Impedance Tomography (EIT) is an imaging technique that is based on the application of alternating electrical signals, whose frequencies range between 10 kHz and 2.5 MHz, on the patient's body surface. The equipment used for this purpose comprises a plurality of sensors (electrodes) placed in contact with the skin, which are connected, by means of electrical conductors, to a processing unit which produces the said alternate signal. The method used comprises a plurality of steps wherein in each step a pair of electrodes is selected for injecting the said signal, while induced voltages, which are captured by the non-selected electrodes, are measured. In the subsequent steps, other pairs of electrodes are selected for injecting the signal, this sequence continuing until all the electrodes of the equipment have been selected, thereby completing an exploration cycle. The induced voltages captured by the electrodes are submitted to treatment by a specific software, allowing the generation of images that represent ventilation and perfusion phenomena in the body of interest.

In research environments, it is acceptable that said electrodes be individually placed around the patient's chest. However, this approach is difficult and depends on the user, who must be attentive to maintain proper alignment of the positioned electrodes. In order to solve this issue, different solutions to mount the electrodes in modules of easy application to the patient have already been described and developed.

The impedance signals captured at each cycle include the signals resulting from factors originated by the normal functioning of the organism. Accordingly, for instance, the signals captured by the electrodes in each measuring cycle will undergo distortions due to the influence of the electrical and mechanical signals produced by the cardiac activity, and as such, this influence must be separated from the signal originated by the mechanical ventilation in order to improve the analysis of each one of these phenomena.

Thus, there is a well-known need to synchronize the acquisition of the impedance signal with a device that may supply the Electrocardiogram (ECG) signal, so that the algorithm of the EIT device may be able to identify the instant of the heartbeat and, thus, separate the effect of this activity from the ventilation activity. Alternatively, the device providing the ECG signal can also indicate the instant in which the heartbeat occurs, as well as other relevant instants of the cardiac cycle.

This integration with the ECG device can be achieved through different methods.

For instance, according to a fist method, the ECG device can be an equipment different from the EIT equipment or it can be part of a different equipment (such as a multi-parameter vital signs monitor, common in intensive care units, operating rooms and emergency rooms).

In this case, there is a drawback with regard to integration, since hospital environments usually possess equipment of different brands, as the integration of the ECG equipment with the EIT equipment demands both the coordination between different providers and the alignment of their interest.

According to a second method, for instance, the ECG device can be incorporated into the EIT device. This solution is also well known and solves the problem of the integration between different devices that usually originate from different suppliers. The circuit for the acquisition of the ECG can either be placed on an electronic board independent from the EIT circuit or it may be integrated into the EIT circuit itself, sharing the use of some of its electronic components.

Although this second method solves the main drawback of the first method, it entails a major problem, which is an excess of electrodes applied to the patient. In this case, the patient for whom the EIT device is installed should have two sets of electrodes for the acquisition of ECG signals applied to his/her chest, wherein: the first one is intended for use by the EIT device; and the second is to be used by the ECG monitor, which can be either an independent equipment or a portion of a multi-parameter vital signs monitor.

Furthermore, another drawback caused by this excess of electrodes is the difficulty of use of the EIT device itself, in addition to the significant discomfort suffered by the patient.

These sets of electrodes for ECG acquisition usually comprise 3, 5 or 10 cables, which are concentrated in an intermediate connecting component from which a thicker cable containing all the signals leads to a connector in the ECG device.

The same issue exists when the EIT device is a module of a multi-parameter vital signs monitor, entailing a worse issue since that in this case there is less freedom for positioning the EIT device to facilitate the application of the electrodes. Although synchronism with the ECG acquisition module is facilitated by the fact of being from the same manufacturer, it is not suitable, as it usually takes place by sending to the EIT module the cardiac events identified only upon a digital analysis of the electrocardiogram signal, which implies delays that are not necessarily uniform.

Apart from the aforementioned examples, some documents reflecting the current state of the art are worth mentioning.

In this regard, reference is made to document PI 0704408-9 which describes modular belts provided with a plurality of electrodes, intended for the application around a segment of the body of a patient or even an animal, although, it does not refer to the addition of electrocardiogram electrodes to the same belt.

On the other hand, document PI 0805365 describes electrodes used to apply transdermal electrical stimuli to patients and/or capture patients' electrical signals, with the aim of providing a solution both economic and of easy application to the patient. Yet, this document also fails to describe the inclusion of electrocardiogram electrodes and, thus, it does not solve the problems of integration with an electrocardiogram device.

Document US 2006/0058600 describes electrodes mounted on modular belts that are longitudinally coupled by means of fittings that allow obtaining sets with various amounts of electrodes. However, this document does not refer to the acquisition of the electrocardiogram signal by means of this belt.

Document US 4,722,354 describes a conductive deformable knitted fabric that is impregnated with a conductive adhesive that adheres to the skin of the patient. The electrical contact is provided by a flexible multi-conductor cable, and the insulation at the end of the cable is removed to allow the separation of these conductors on the surface of said fabric. An insulating plastic plate is glued on this set to avoid accidental electrical contact with the conductive portions of the electrode.

Document US 4,736,752 describes that the conductive portion of the electrode consists of a plurality of conductive paint traces applied on an insulating flexible base such as a thin sheet of polyethylene.

The above commented documents refer to constructive aspects of belt-shaped modules to capture body signals and to the electrodes, without solving the problem entailed by the current belts.

Lastly, document PI 0801014-5 refers to the use of data, signals, events and information captured by different means and equipment in order to improve the function of the electrical impedance tomography. This document considers the case in which the ECG device can be incorporated into the EIT device, but does not solve the important practical problem of the application of two sets of electrocardiogram electrodes.

Thus, in order to solve the problems identified above and other problems existing in the prior art, one of the objective of this invention consists in the provision of a module to capture body signals comprising sensors dedicated to the capture of electric signals from the heart, these sensors being arranged on the same surface of the module.

One other of the objectives of the invention consists in the provision of a module to capture body signals comprising sensors that simultaneously capture cardiac electric signals end body impedance signals, such sensors being arranged on the same side of the module.

Through its particular characteristics, the present invention can further solve other problems of the prior art which have not been brought up herein, since the list reported herein of devices and methods to capture body signals and their problems is exemplary and not exhaustive.

### DISCLOSURE

In order to circumvent the foregoing drawbacks of the prior art, among others, the present invention refers to a module to capture body signals that comprises at least one sensor to capture cardiac electrical signals and at least one sensor to capture body impedance signals.

In the same context, the present invention also refers to a module for capturing body signals, which comprises at least one sensor capable of simultaneously capturing cardiac electric signals and/or body impedance signals.

In conformity with additional or alternative embodiments of the invention, the following characteristics, and their possible variants, can also be present, alone or in combination:
- heart electrical signals are electrocardiogram signals;
- body impedance signals are signals used for electrical impedance tomography;
- sensors for heart electrical signals and body impedance are electrodes;
- the sensor for simultaneous capture is an electrode;
- the module comprises a first surface and a second surface, wherein the first surface is opposite to the second surface and wherein the first surface is capable of being in contact with the body;
- the at least one sensor to capture heart electrical signals and at the least one sensor to capture body impedance signals are placed on the first surface;
- the at least one simultaneous sensor is placed on the first surface;
- the module has an elongated shape; and
- the module is made of flexible material.

### BRIEF DESCRIPTION OF DRAWINGS

The objectives, functional improvements and advantages of the module to capture body signals that constitute the object of the present invention will be evident to technicians skilled in the art from the following description of a particular embodiment, which refers to the attached figures. These figures are schematic and their sizes and proportions may not correspond to reality, since they only aim at describing the invention in a didactical fashion, without imposing any limitations beyond from those defined by the claims described further on:
Figure 1 is a perspective view of a first embodiment of the invention; and
Figure 2 is a perspective view of a second embodiment of the invention.

### MODE(S) OF CARRYING OUT THE INVENTION

The invention will now be described with relation to the particular embodiments thereof, with reference being made to the attached figures. In the following figures and description, similar parts are indicated along the specification and figures with identical reference numbers. The figures are not necessarily drawn to scale. Certain characteristics may be over-scaled or in a rather schematic fashion, and some details of conventional elements may not be represented, for better clarity and conciseness of this description. The invention allows different embodiments. Particular embodiments are described in detail and shown in the figures, and they should be deemed to constitute an exemplification of the principles of the invention, without the intent of limiting the invention to what is merely illustrated and described in this specification. It should be acknowledged that the different teachings of the embodiments discussed below may be used separately or in any suitable combination to produce the same desired effects.

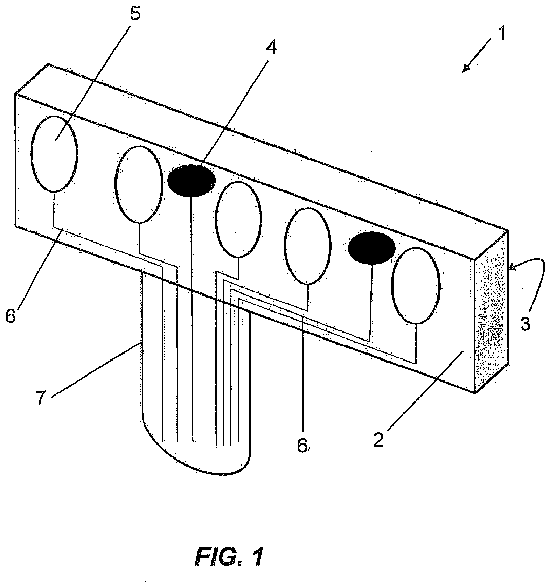

Figure 1 shows a first embodiment of the invention. According to this first embodiment, module 1 is elongated and comprises two surfaces, wherein a first surface 2 is configured to be in contact with the body of a patient (human or animal) and a second surface 3 is opposite to the first surface 2. The shape of each one of the surfaces 2 and 3 may vary within the scope of the invention so that the substantially rectangular shape shown in Figure 1 may be different, depending on how the present invention will be realized. Thus, in alternative embodiments, for instance, the first surface 2 may exhibit a rectangular shape with rounded corners, or even surfaces comprising undulations along the outline thereof.

Furthermore, in order to facilitate its application to the body of a patient, whether human or animal, the module 1 is made of a flexible material such as, for example, a polymeric material with elastic properties. Additionally, the module 1 may be shaped as a belt, which can be combined with the flexibility afforded by the said polymeric material, easing the application and a greater degree of comfort to the patient.

Referring again to Figure 1, it is noted that the module comprises two types of sensors, with at least one sensor intended to capture cardiac electrical signals 4 and at least one sensor to capture body impedance signals 5, and these sensors 4 and 5 are placed on the first surface 2 of the module. Therefore, it is noted that sensors 4 and 5 are arranged in the same area or, in other words, on the same "footprint".

In particular, cardiac electrical signals are electrocardiogram (ECG) signals and body impedance signals are used for electrical impedance tomography (EIT). Moreover, in a particular embodiment of the present invention, the sensors for cardiac electrical signals 4 and for body impedance signals 5 are electrodes.

Particularly, module 1 may comprise from 8 to 32 sensors to capture body impedance signals 5, arranged on the first surface 2. It is important to point out that the extra amount of sensors to capture impedance signals 5 may vary depending on the embodiment of the invention. Moreover, the arrangement of these sensors 5 may conform to a spacing and distribution pattern, that is, these sensors may be provided in alignment or not and with predetermined distances between one another.

Also particularly, the module may comprise a variable amount of sensors to capture cardiac signals 4, wherein the arrangement of these sensors 4 on the first surface of module 1 may or not conform to an arrangement pattern. Hence, these sensors 4 can be arranged in random positions, at the discretion of whoever will realize the invention.

Accordingly, in view of the aforementioned layout and arrangement options, the present invention may be realized with the sensors to capture cardiac electrical signals 4 arranged between the sensors for capture of impedance signals 5, or even in another region of the first surface 2.

Thus, as module 1 already comprises in its body (on the first surface 2, to be more precise) sensors to capture cardiac electrical signals 4 and impedance signals 5, there is no need to add, couple or connect additional sensors to measure cardiac electrical signals, such as ECG signals.

Furthermore, the sensors to capture cardiac electrical signals 4 and the sensors to capture body impedance signals 5 may have various shapes and, thus, such feature does not set limitations to the scope of the present invention. Accordingly, for instance, such sensors may exhibit, for example, circular, oblong, rectangular, elliptic shapes, among other possible shapes.

As shown in figure 1, to each sensor 4 and 5 is connected at least one electrical conductor 6, and these electrical conductors 6 converge towards an outlet 7 associated to the body of module 1. It will be worth pointing out at this stage that the representation of electrical conductors 6 in figure 1 has a didactic purpose and, thus, it is schematic, since such conductors 6 are internally provided in the module 1.

The said outlet 7 has a tubular shape and it may be integrally formed with the body of module 1 or may constitute a separate device attached to module 1. Furthermore, this outlet 7 can be provided as a single or main cable ("trunk cable") connected to the body of module 1, which combines the electrical conductors 6 of the various sensors 4 and 5. Additionally, outlet 7, built as a single or main cable, may also comprise a connector (not shown), capable of connecting the electrical conductors 6 of module 1 to: an impedance tomography (EIT) device; a device which integrates the functions of impedance tomography (EIT) and of electrocardiogram (ECG); or other devices to monitor patient conditions which use the data captured by sensors 4 and 5.

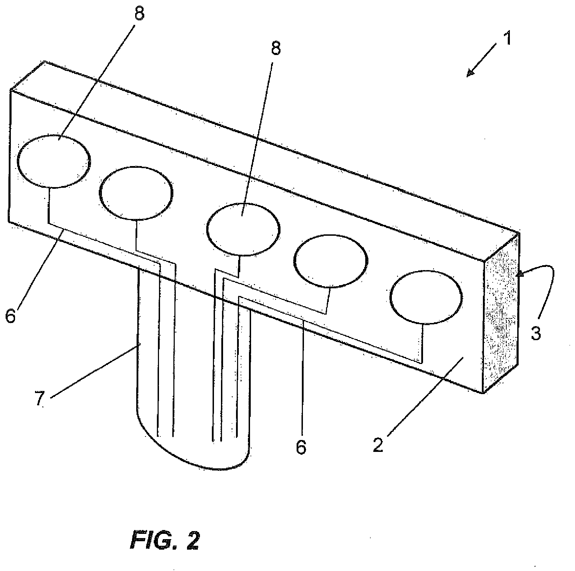

Figure 2 shows a second embodiment of this invention. Module 1 of the second embodiment of the invention has the same characteristics of the first embodiment, except for the fact that it comprises at least one sensor 8 capable of simultaneously capturing cardiac electrical signals and/or body impedance signals. Accordingly, in this second embodiment, module 1 comprises a versatile type of sensor capable of capturing both the cardiac electrical signals and the body's impedance signals.

The shape and arrangement of this simultaneous sensor 8 may also vary, and there can also be more than one sensor 8 provided in alignment or not, such as in the case of sensors 4 and 5 of the first embodiment. Moreover, this sensor 8 may also be realized as an electrode, placed on the first surface 2 of the module 1, that is, in the same area or "footprint", such as already described for sensors 4 and 5 of the first embodiment of the invention. Thus, as the module 1 already comprises in its body (on the first surface 2, to be more precise) a sensor to capture cardiac electrical signals and impedance signals, there is no need to add, couple or connect additional sensors to measure cardiac electrical signals, such as the ECG signals.

The exact amount of simultaneous sensors 8 arranged on the first surface 2 of module 1 may also vary within the scope of the invention. Such as occurs in the first embodiment, module 1 of the second embodiment of the invention may comprise from 8 to 32 simultaneous sensors 8.

Obviously, these figures represent an example of quantity and, therefore, they are not exacting or limiting with respect to the scope of the invention.

Additionally, as schematically shown in figure 2, to each simultaneous sensor 8 there is connected at least one electrical conductor 6, such as occurs in the first embodiment of the invention. These electrical conductors 6 also converge towards an outlet 7, which can be realized as a single or main cable ("trunk cable") connected to an impedance tomography (EIT) device; a device that comprises the functions of impedance tomography (EIT) and of electrocardiogram (ECG); an electrocardiogram device; or other devices to monitor patient conditions which use the data captured by the simultaneous sensor 8.

Another difference that is worth pointing out with respect to the first embodiment of the invention resides in the fact that the apparatus that receives the signals captured by the simultaneous sensor 8 comprises means to identify and separate cardiac electrical signals and body impedance signals.

In particular, these means to identify and separate signals captured by the simultaneous sensor 8 may comprise amplifiers and filters. Thus, for instance, amplifiers with high input impedance may be connected to the main cable itself or, more precisely, to electrical conductors 6. Upon passing through the amplifier, a high-pass filter may be applied in order to eliminate sensor offset.

This high-pass filter may be of first order with 2Hz cutoff frequency. Subsequently, there may also be applied a low-pass filter in order to eliminate the high frequency of the power supply of the device and the one generated upon sensor scanning, which is performed to generate images. This low-pass filter may be of eight order, with150 gain, and with cutoff frequency at 40 Hz. At the end of these amplification and filtration steps, the cardiac electrical signal is obtained.

As may be noted, the arrangement of the sensors to capture cardiac electrical signals 4 and sensors to capture body impedance signals 5 on the same surface 2 (or "footprint") of module 1, brings considerable advantages to the present invention. Among these advantages, there may be cited the fact that there is no need for additional or coupled sensors to measure cardiac electrical signals, such as electrocardiogram signals. Thus, the prior art problem of excess of electrodes is eliminated.

Furthermore, another advantage is associated with the fact that the electrical conductors 6 converge towards an outlet 7, which is directly associated to the body of module 1 and realized as a single or main cable ("trunk cable") connected to the patient monitoring device. Among the advantages, it may be cited that the patient and the medical staff cease to be hindered by the large amount of wires and cable, if we remember that to each prior art electrode there is connected one wire or cable. It is further worth remembering that these prior art wires and cables were placed on the hospital bed, on the patient, or even between the bed and the apparatus to monitor the patient's condition. The present invention successfully eliminates this problem.

It is also worth mentioning the synergistic effect existing by combining sensors 4, 5 or 8 on the same surface 2 (or "footprint") of module 1 with the convergence of the electrical conductors towards an outlet 7 realized in the form of a single or main cable. The practical characteristic and ease of use brought by the combination of these two factors make module 1 obviously advantageous and inventive with respect to the prior art.

Additionally, it will be worth pointing out that the second embodiment of this invention brings advantages regarding versatility. More precisely, given that the simultaneous sensors 8 can capture cardiac electrical signals and/or body impedance signals at the same time, the device to monitor the patient can be configured to use such electrode 8 as an electrocardiogram electrode, an impedance tomography electrode or both. Consequently, a certain amount of simultaneous sensors 8 may be configured to operate as an electrocardiogram electrode and another certain amount may be configured to operate as impedance tomography electrode.

Although the module to capture body signals of the present invention is particularly useful to capture cardiac electrical signals and body impedance signals, the module of this invention may be built for other types of applications and may be modified in the manner in which it is implemented, so that the scope of protection of the invention is limited only by the content of the attached claims, including the possible equivalent variations thereof.

## Claims

1. A MODULE (1) TO CAPTURE BODY SIGNALS **characterized in that** it comprises at least one sensor to capture cardiac electrical signals (4) and at least one sensor to capture body impedance signals (5).

2. A MODULE (1), according to claim 1, **characterized in that** the cardiac electrical signals are electrocardiogram signals and the body impedance signals are used for electrical impedance tomography.

3. A MODULE (1), according to claim 1, **characterized in that** the sensors for cardiac electrical signals and body impedance signals are electrodes.

4. A MODULE (1), according to claim 1, **characterized in that** it comprises a first surface (2) and a second surface (3), wherein the first surface (2) is provided opposite to the second surface (3) and capable of being in contact with the body, and further **in that** at least one sensor to capture cardiac electrical signals (4) and the at least one sensor to capture body impedance signals (5) are arranged on the first surface (2).

5. A MODULE (1), according to claim 1, **characterized in that** it has an elongated shape and is made of a flexible material.

6. A MODULE (1) TO CAPTURE BODY SIGNALS **characterized in that** it comprises at least one sensor (8) capable of simultaneously capturing cardiac electrical signals and/or body impedance signals.

7. A MODULE (1), according to claim 6, **characterized in that** the cardiac electrical signals are electrocardiogram signals and the body impedance signals are signals used for electrical impedance tomography.

8. A MODULE (1), according to claim 6, **characterized in that** simultaneous sensor (8) is an electrode.

9. A MODULE (1), according to claim 6, **characterized in that** it comprises a first surface (2) and a second surface (3), wherein the first surface (2) is provided opposite to the second surface (3) and is capable of being in contact with the body, and further **in that** at least one simultaneous sensor (8) is arranged on the first surface (2).

10. A MODULE (1), according to claim 6, **characterized in that** it has an elongated shape and is made of a flexible material.
